# EUROPEAN PATENT SPECIFICATION

(11) **EP 0 816 347 B1**
(45) Date of publication and mention of the grant of the patent: **20.06.2007**
(21) Application number: 97304498.5
(22) Date of filing: 25.06.1997
(51) Int. Cl.: C07D 263/48, C07D 207/40

(54) **Ammonium oxazole- and amino oxazolium intermediates, methods for their preparation and their use in the manufacture of insecticidal, acaricidal and nematocidal arylpyrrole derivatives**
Ammonium-Oxazol- und Amino-Oxazolium Zwischenprodukte, Verfahren zu deren Herstellung und deren Verwendung in der Herstellung von insektiziden, akariziden und nematoziden Arylpyrrol Derivaten
Produits intermédiaires ammonium oxazole et amino oxazolium, procédé pour leur préparation et leur utilisation pour la préparation de dérivés arylpyrrole insecticides, acaricides et nématocides

(30) Priority: 28.06.1996 US 672787
(43) Date of publication of application: 07.01.1998
(73) Proprietor: BASF Aktiengesellschaft, 67056 Ludwigshafen (DE)
(72) Inventor: Kameswaran, Venkataraman, Princeton Junction, New Jersey 08550 (US)
(74) Representative: Pohl, Michael Friedrich

(56) References cited:
- EP-A- 0 713 868
- CHEMICAL ABSTRACTS, vol. 78, no. 7, 19 February 1973 Columbus, Ohio, US; abstract no. 43335r, POUPAERT J. ET AL.: "N-acyl-.alpha.-aminonitriles in the Pinner reaction" page 478; column 2; XP002044525 & SYNTHESIS , no. 11, 1972, pages 622-624,
- MC EWEN W.E. ET AL.: "Synthetic uses of open-chain analogues of Reissert compounds" THE JOURNAL OF ORGANIC CHEMISTRY, vol. 45, no. 7, 28 March 1980, pages 1301-1308, XP002044524

## Description

Arylpyrrole carbonitrile compounds are highly effective insecticidal, acaricidal and nematocidal agents with a unique mode of action and a broad spectrum of activity. In particular, 2-aryl-5-(trifluoromethyl)pyrrole-3-carbonitrile compounds demonstrate effective control across a wide array of pests and can control resistant pests such as pyrethroid-, organophosphate-, cyclodiene-, organo-chlorine-, organotin-, carbamate-, and benzophenyl-urea-resistant biotypes of *Helicoverpa*/*Heliothis* spp., *Spodoptera* spp.*, Trichoplusia* spp., *Pseudoplusia* spp. and *Tetranychus* spp.. Because there is no apparent cross-resistance, 2-aryl-5-(trifluoromethyl)pyrrole-3-carbonitrile compounds and their derivatives have potential for use in resistance management programs. Further, said pyrroles have little effect on beneficial species making them excellent candidates for integrated pest management programs, as well. These programs are essential in today's crop production.

Therefore, methods to prepare said pyrroles and intermediates to facilitate their manufacture are of great use. Among the present methods to prepare 2-aryl-5-(trifluoromethyl)pyrrole-3-carbonitrile on a manufacturing scale are the 1,3-dipolar cycloaddition of 3-oxazolin-5-one with 2-chloroacrylonitrile (U. S. 5,030,735) and the cycloaddition reaction of the appropriate oxazole amine derivatives with 2-chloroacrylonitrile or 2,3-dichloropropionitrile (U.S. 5,446,170).

Also known is the 1,3-dipolar cycloaddition of the mesionic intermediate product of the acid catalyzed cyclization of a Reissert compound with a suitable alkyne to give an N-substituted pyrrole product as described by W. M. McEwen, et al, Journal of Organic Chemistry, 1980, 45, 1301-1308. However these mesionic intermediates undergo 1,4-cycloaddition reactions with ethylenic dieneophiles to give an aroylpyrrole derivative and, as such, are not useful as insecticidal arylpyrrole precursors.

Therefore, it is an object of this invention to provide a source of important intermediate ammonium oxazole and amino oxazolium compounds useful in the manufacture of arylpyrrole pesticidal agents.

It is another object of this invention to provide a method to prepare said intermediate compounds.

It is an advantage of this invention that said intermediates may be utilized in a manufacturing process which produces a formula I arylpyrrole precursor capable of being converted to a wide variety of highly effective insecticidal, acaricidal and nematocidal agents.

It is a feature of this invention that said process provides a regiospecific product. These and other features and objects of the invention will become more apparent from the detailed description set forth hereinbelow.

The present invention provides an ammonium oxazole intermediate of formula I, 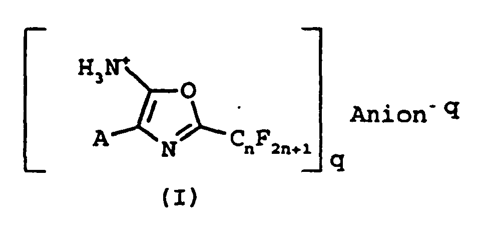 wherein n is an integer of 1, 2, 3, 4, 5, 6, 7 or 8; q is an integer of 1, 2, or 3; A is 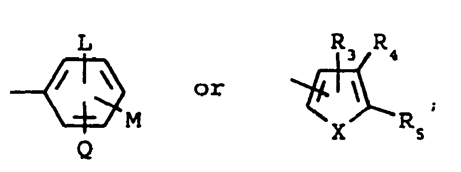 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure

-OCH₂O-, -OCF₂O- or -CH=CH-CH=CH-;

R₁ and R₂ are each independently C₁-C₄alkyl;
R₃, R₄, and R₅ are each independently hydrogen, halogen, NO₂, CHO or R₄ and R₅ may be taken together with the atoms to which they are attached to form a ring in which R₄R₅ is represented by the structure 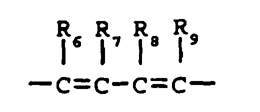 R₆, R₇, R₈ and R₉ are each independently hydrogen, halogen, CN or NO₂;
X is O or S; and
Anion^{-q} is a proton acceptor having a negative q charge; or
the tautomers thereof.

The present invention also provides an oxazolium intermediate of formula II, or the tautomers thereof, 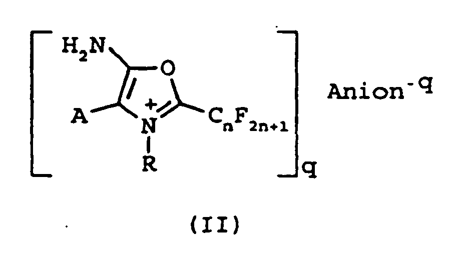 wherein n, q, A and Anion^{-q} is as described hereinabove for formula I and R is C₁-C₆alkyl optionally substituted with one C₁-C₄alkoxy or phenyl group.

Further provided are methods for the preparation of the compounds of formula I and formula II and their use in the manufacture of insecticidal, acaricidal and nematocidal arylpyrrole compounds.

Processes, to be useful on a manufacturing scale, preferentially contain key intermediate compounds which may be obtained in high to quantitative yield, which are stable either upon isolation or *in situ,* which may be produced from simple or readily available starting materials and which may be readily converted to the desired end-product of manufacture in a minimum of reaction steps, in optimum yield and purity and, if applicable, regio- or stereospecifically.

It has now been found that 5-ammonium oxazole intermediates of formula I and 5-amino oxazolium intermediates of formula II and tautomers thereof are effective intermediates in the manufacture of 2-aryl-5-(perfluoroalkyl)pyrrole-3-carbonitrile insecticidal, acaricidal and nematocidal agents.

The ammonium oxazole and amino oxazolium compounds of the invention have the structure of formula I and formula II, respectively 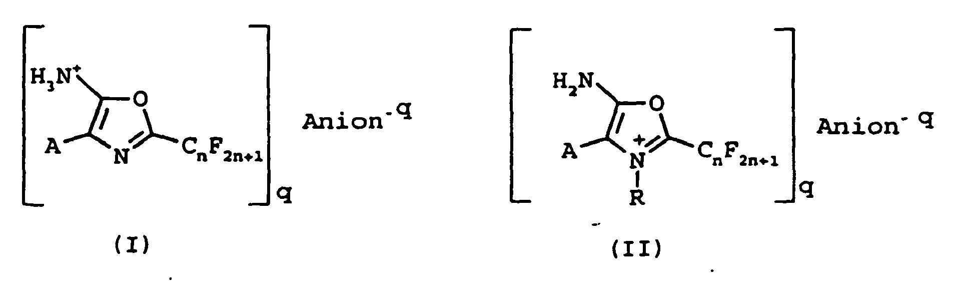 wherein n is an integer of 1, 2, 3, 4, 5, 6, 7 or 8;
q is an integer of 1, 2, or 3; A is 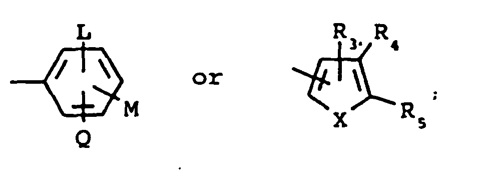 L is hydrogen or halogen;
R is C₁-C₆alkyl optionally substituted with one alkoxy or phenyl group;
M and Q are each independently hydrogen, halogen, CN, NO₂, C₁-C₄alkyl, C₁-C₄haloalkyl, C₁-C₄alkoxy,
C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure

-OCH₂O-, -OCF₂O- or -CH=CH-CH=CH-;

R₁ and R₂ are each independently C₁-C₄alkyl;
R₃, R₄, and R₅ are each independently hydrogen, halogen, NO₂, CHO or R₄ and R₅ may be taken together with the atoms to which they are attached to form a ring in which R₄R₅ is represented by the structure 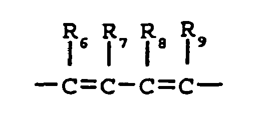 R₆, R₇, R₈ and R₉ are each independently hydrogen, halogen, CN or NO₂;
X is O or S; and
Anion^{-q} is a proton acceptor having a negative q charge; or
the tautomers thereof.

The term halogen as used in the specification and claims designates Cl, Br, F or I and the term haloalkyl embraces any alkyl group of χ carbon atoms which may contain from 1 to 2χ+1 halogen atoms which may be the same or different.

The 5-ammonium oxazole intermediate of formula I and the 5-amino oxazolium intermediate of formula II may be represented by their respective tautomeric structures Ia and IIa. Said tautomers are shown below wherein n, q, A, R and Anion^{-q} are as described hereinabove.

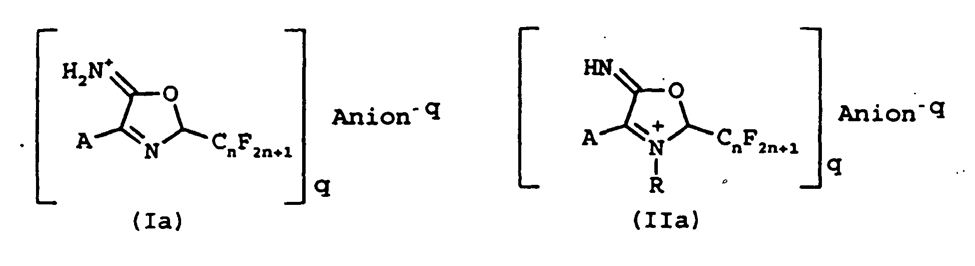

The formula I and formula II compounds of the invention are preferably those compounds wherein q is 1 or 2, with q=1 being especially preferred. Formula I compounds include but are not limited to
5-amino-4-(*p*-chlorophenyl)-2-(trifluoromethyl)-oxazole, methanesulfonic acid salt;
5-amino-4-(*p*-chlorophenyl)-2-(trifluoromethyl) oxazole, *p*-toluenesulfonic acid salt;
5-amino-4-(3,5-dichlorophenyl)-2-(trifluoromethyl) oxazole, trifluoromethanesulfonic acid salt;
5-amino-4-(3,5-dichlorophenyl)-2-(trifluoromethyl) oxazole, methanesulfonic acid salt;
5-amino-4-[*p*-(α,α,α-trifluoro)tolyl]-2-(trifluoromethyl)oxazole, *p*-toluenesulfonic acid salt;
5-amino-4-(3,4,5-trichlorophenyl)-2-(trifluoromethyl)oxazole, *p*-toluenesulfonic acid salt;
5-amino-4-[*p*-(trifluoromethoxy)phenyl]-2-(trifluoromethyl)oxazole, trifluoromethanesulfonic acid salt; or
5-amino-4-[2- (3,4,5-trichloro)thienyl]-2-(trifluoromethyl)oxazole, methanesulfonic acid salt.

The formula II compounds of the invention include but are not limited to
5-amino-4-(*p*-chlorophenyl)-3-methyl-2-(trifluoromethyl)oxazalium tetrafluoroborate;
5-amino-4-(*p*-bromophenyl)-3-methyl-2-(trifluoromethyl)oxazolium methanesulfonate;
5-amino-4-(3,5-dichlorophenyl)-3-benzyl-2-(trifluoromethyl)oxazolium *p*-chlorotoluenesulfonate;
5-amino-4-(3,4,5-trichlorophenyl)-3-isopropyl-2-(trifluoromethyl)oxazolium tetrafluoroborate;
5-amino-4-(*p*-chlorophenyl)-3-methyl-2-(trifluoromethyl)oxazolium bisulfate;
5-amino-4-[(α,α,α-trifluoro)tolyl]-3-benzyl-2-(trifluoromethyl)oxazolium *p*-chlorotoluenesulfonate;
5-amino-4-[*p*-(trifluoromethoxy)phenyl]-3-ethyl-2-(trifluoromethyl)oxazolium tetrafluoroborate; or
5-amino-4-[2-(3,4,5-trichloro)thienyl]-2-(trifluoromethyl)oxazolium methanesulfonate.

Advantageously, the 5-ammonium oxazole intermediates of formula I or the 5-amino oxazolium intermediates of formula II, may be obtained by cyclizing a formula III amide nitrile in the presence of an acid and a solvent under essentially anhydrous conditions. The reaction is shown in flow diagram I wherein q is 1, R' is hydrogen or

R and n, A, R and Anion⁻ are as described hereinabove.

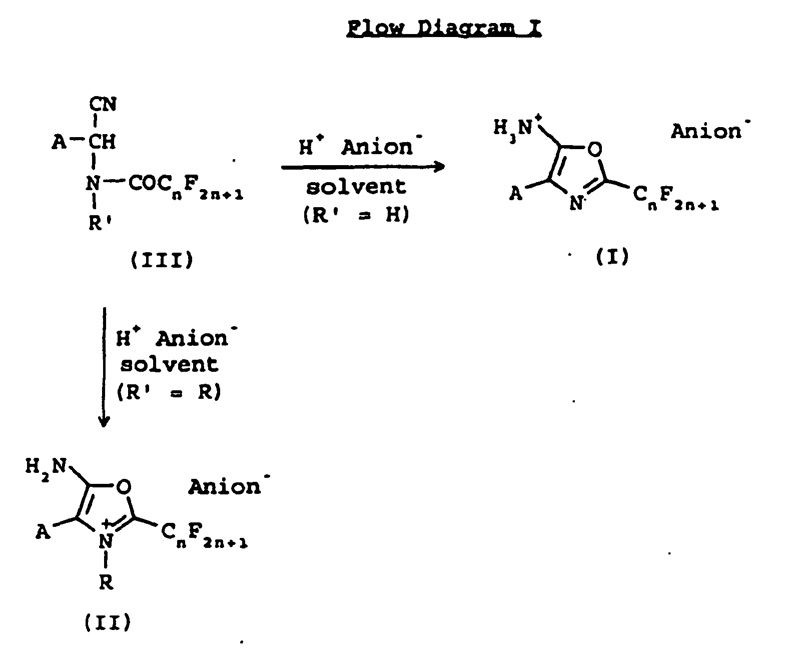

The formula I and formula II intermediates may be isolated by conventional means such as filtration or chromatographic separation and serve as an expedient source of a key intermediate for insecticidal pyrrole manufacture or for future derivatization.

Solvents suitable for use in the above reaction are those organic solvents capable of sustaining essentially anhydrous conditions and partial or complete dissolution of the amide nitrile compound of formula III. Said solvents include: aromatic hydrocarbons such as benzene, xylene, toluene, preferably toluene; chlorinated aromatic hydrocarbons such as chlorobenzene; carboxylic acid amides such as dimethylformamide, N-methylpyrrolidone, preferably dimethylformamide; nitriles such as acetonitrile, propionitrile; alcohols such as isopropanol, *t*-butanol, *sec-*butanol, preferably *t*-butanol. These solvents may be used alone or in combination of two or more.

Acids suitable for use in the formation of the compounds of the invention are any acids capable of relative dehydration. Among the suitable acids are sulfuric acid, methanesulfonic acid, trifluoromethane sulfonic acid, *p*-toluenesulfonic acid, phosphoric acid, tetrafluoroboric acid, tetrafluoroboric acid complexes. Boron trifluoride complexes such as boron trifluoride acetic acid, boron trifluoride dihydrate are also suitable acids.

The formula III amide nitrile compounds wherein R' is hydrogen and their preparation are described in U.S. 5,426,225. Formula III amide nitrile compounds wherein R' is R may be obtained via the perfluoroacylation of the appropriate amino nitrile of formula IV. The formula IV aminonitriles are correspondingly readily obtained from their available benzaldehyde, furfurylaldehyde or thienylmethylaldehyde precursors via the well-known Strecker reaction. The reaction sequence is shown in Flow Diagram II wherein n, A and R are as described hereinabove for formulas I and II, m is an integer of 1 or 2, X₁ is Cl, OR₁₀ or O and R₁₀ is hydrogen or C₁-C₆alkyl with the proviso that when X₁ is O, then m must be 2 and when X₁ is Cl or OR₁₀, then m must be 1.

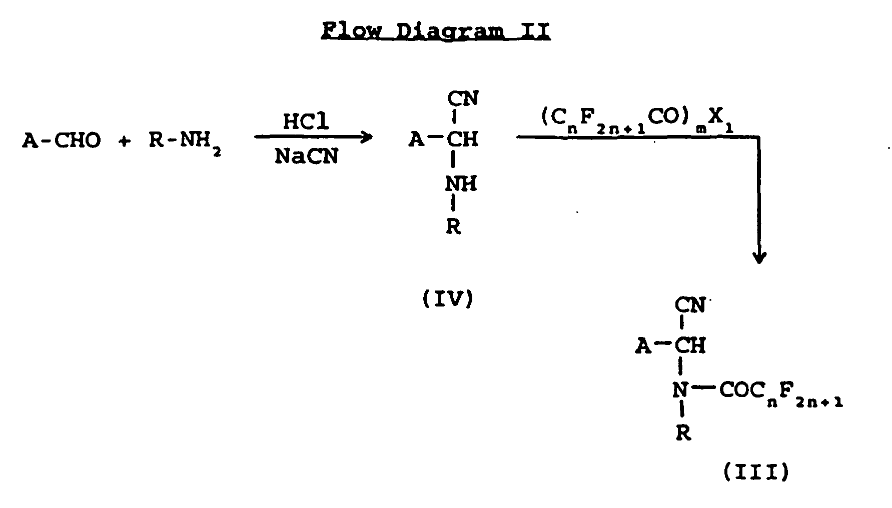

Advantageously, the formula I and II intermediates may be converted to 2-aryl-5-perfluoroalkylpyrroles of formula IV by the 1,3-dipolar cycloaddition of at least one molar equivalent of a dieneophile of formula V in the presence of a solvent and essentially in the absence of water. The conversion is illustrated in Flow Diagram III wherein n, A and R are described hereinabove and q is 1, W is CN, NO₂, COOR₁, or COR₂; R₁ and R₂ are each independently C₁-C₄alkyl; and Y is H, Cl or Br with the proviso that when the intermediate compound is formula I then Y must be Cl or Br.

Particular compounds of formula (IV) of interest are those where W is CN, R is methyl, n is 1 or 2, and A is phenyl optionally substituted with halogen, C₁-C₄haloalkyl, or C₁-C₄haloalkoxy.

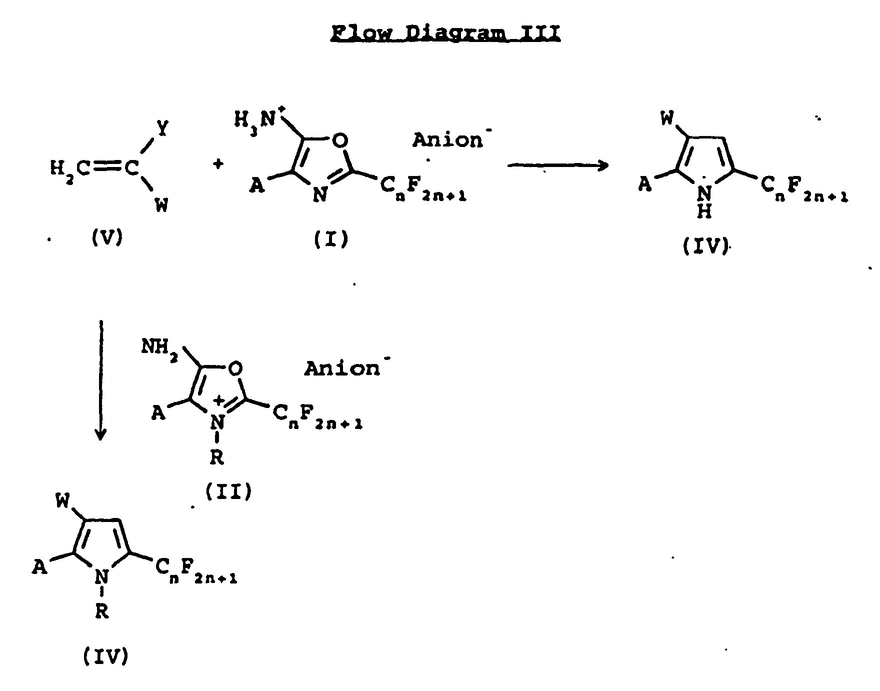

Solvents suitable for use in the above reaction are those organic solvents capable of sustaining essentially anhydrous conditions and partial or complete dissolution of the amide nitrile compound of formula III. To the extent water is present, lower product yield and decreased purity is expected. Said solvents include: aromatic hydrocarbons such as benzene, xylene, toluene, preferably toluene; chlorinated aromatic hydrocarbons such as chlorobenzene; carboxylic acid amides such as dimethylformamide, N-methylpyrrolidone, preferably dimethylformamide; nitriles such as acetonitrile, propionitrile; alcohols such as isopropanol, *t*-butanol, *sec*-butanol preferably *t*-butanol. These solvents may be used alone or in combination of two or more.

While the formula IV compounds have insecticidal activity, their greatest utility may be as precursors to certain formula IVa compounds: 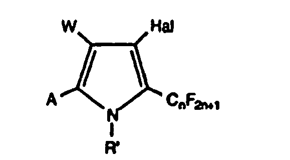 wherein W, A, R' and n are as hereinabove defined and Hal is halogen.

Advantageously, the process of the invention allows the preparation of formula IVa 2-aryl-4-halo-5-(perfluoroalkyl)pyrrole-3-carbonitrile insecticidal, acaricidal and nematicidal agents by the 1,3-dipolar cycloaddition of a dienophile of formula followed by halogenation.

Halogenation methods may be any known methods such as those described in U.S. 5,010,098 or U. S. 5,449,789.

In order to provide a more clear understanding of the invention, the following examples are set forth below.

The terms ¹H NMR, ¹³C NMR and ¹⁹F NMR designate proton, carbon 13 and fluorine 19 nuclear magnetic resonance, respectively. The term HPLC designates high performance liquid chromatography.

### EXAMPLE 1

### Preparation of N-Isopropylamino(p-chlorophenyl)acetonitrile

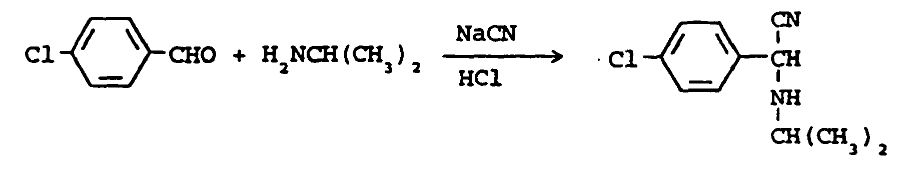

Isopropylamine (88.7g, 1.5 mol) is added to an aqueous solution of concentrated hydrochloric acid (125 mL, 1.5 mL) in water at 25°-30°C. The resultant mixture is treated sequentially with a solution of sodium cyanide (53.9g, 1.1 mol) in water and methylene chloride at 30°C, warmed to 35°C, treated with a solution of p-chlorobenzaldehyde (140.6g, 1 mol) in methylene chloride over a 15-25 minute period, allowed to warm, held for 3 hours at 45°C and cooled to room temperature. The phases are separated and the organic phase is washed with water and concentrated *in vacuo* to give a residue. The residue is crystallized from heptane to give the title product as a pale yellow crystalline solid, 190.3g (91.2% yield), mp 72.0-73.0°C, identified by ¹H and ¹³C NMR analyses.

### EXAMPLE 2

### Preparation of N-(p-chloro-α-cyanobenzyl)-2,2,2-tri-fluoro-N-isopropylacetamide

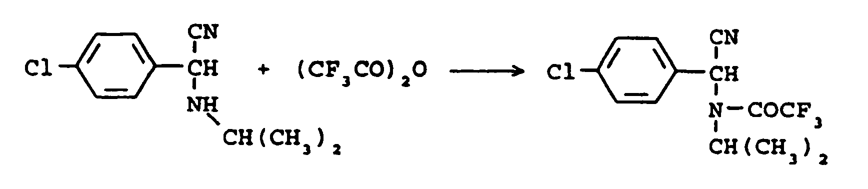

A slurry of N-isopropylamino(p-chlorophenyl)acetonitrile (25.0g, 0.12 mol) in trifluoroacetic anhydride is gently heated at reflux temperature for 20 hours and concentrated *in vacuo* to give an oil residue. The oil is crystallized from toluene/heptane to give the title product as a white solid, 26.5g (72.4% yield) mp 78.5-79.5°C, identified by ¹H, ¹³C and ¹⁹F NMR analyses.

### EXAMPLE 3

### Preparation of N-Benzyl-N-(p-chloro-α-cyanobenzyl)-2,2,2-trifluoroacetamide

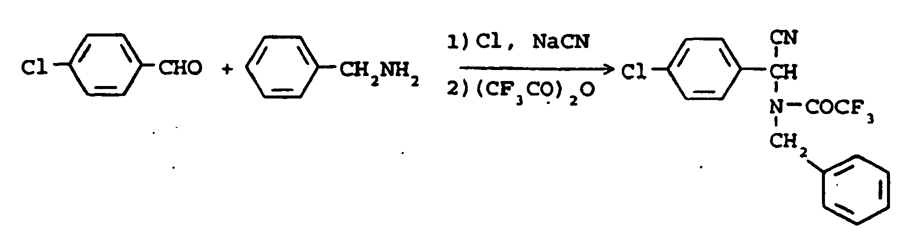

Aqueous hydrochloric acid (62.5 mL of 12 N, 0.75 mol) in water (100 mL) is treated with benzylamine (80.4g, 0.75 mol) at <20°C, then treated sequentially with a solution of sodium cyanide (27.0g, 0.55 mol) in water and methylene chloride, warmed to 35°C, treated with a solution of *p*-chlorobenzaldehyde (70.3g, 0.5 mol) in methylene chloride, allowed to warm to 50°C, and held at 45°C for 3.5 hours. The phases are separated and the organic phase is washed with water and concentrated to a syrup residue. The residue is dissolved in toluene and ethyl acetate, treated with trifluoroacetic anhydride (105.0g, 0.5 mol) at 20°-30°C over a 30 minute period and diluted with heptane. The resultant white fluffy solid precipitate is filtered and dried to give the title product, 119.8g (70.7% yield), mp 131-132°C, identified by ¹H, ¹³C and ¹⁹F NMR analyses.

### EXAMPLE 4

### Preparation of 5-Amino-2-(p-chlorophenyl)-2-(trifluoromethyl)oxazole, trifluoromethanesulfonic acid salt

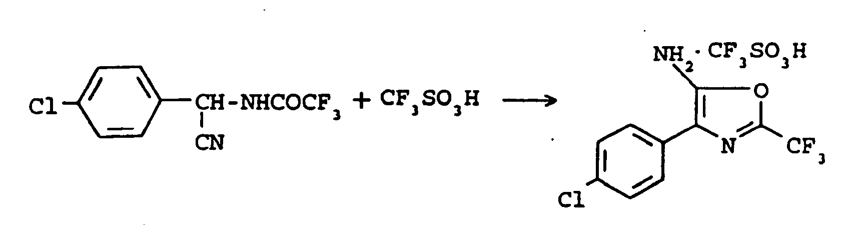

A solution of N-(*p*-chloro-α-cyanobenzyl)-2,2,2-trifluoroacetamide (21.0g, 0.08 mol) in ether is treated at room temperature with trifluoromethanesulfonic acid (24.0g, 0.16 mol) under a nitrogen atmosphere. The ether is slowly evaporated over an 18 hour period to give a pasty solid residue. The residue is treated with ether and filtered and the filtercake is dried to give a yellow solid, 26.7g (81% yield). A small amount of the solid is recrystallized from ethyl acetate to give the title product, mp 147-150°C (dec.), identified by ¹H and ¹⁹F NMR and mass spectral analyses.

### EXAMPLE 5

### Preparation of 5-Amino-2-(p-chlorophenyl)-2-(trifluoromethyl)oxazole, p-toluenesulfonic acid salt

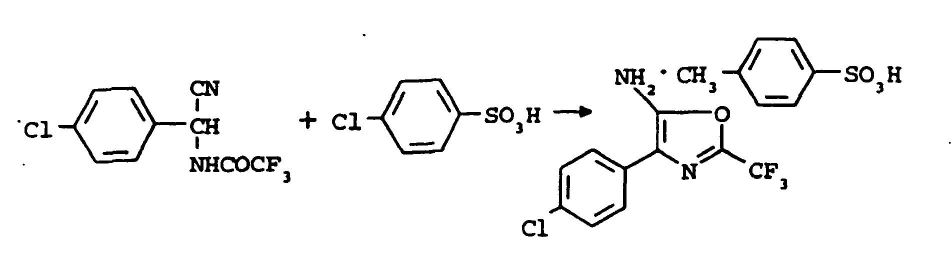

A slurry of *p*-toluenesulfonic acid monohydrate (16.7g, 0.088 mol) in toluene is azeotropically distilled using a Dean-Stark trap to obtain anhydrous acid. The resultant dry toluene solution is cooled, treated with N-(*p*-chloro-α-cyanobenzyl)-2,2,2-trifluoroacetamide (21.0g, 0.08 mol), heated at 100°C for 18 hours, cooled and diluted with ether. The resulting mixture is filtered and the filtercake is crystallized from toluene to give a yellow solid 11.0g (29% yield). A small portion is recrystallized from toluene to give the title product as white needles, mp 164°-165°C, identified by HPLC, ¹H and ¹⁹F NMR and mass spectral analyses.

### EXAMPLE 6

### Preparation of (p-Chlorophenyl)-5-(trifluoromethyl)-pyrrole-3-carbonitrile

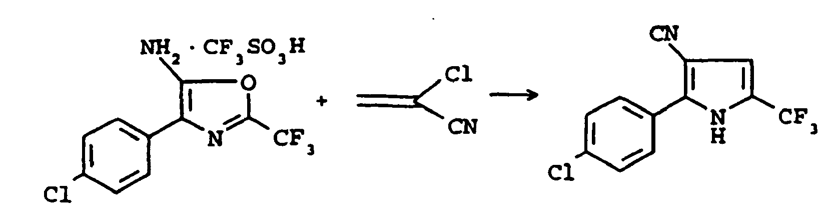

A solution of 5-amino-4-(*p*-chlorophenyl)-2-(trifluoromethyl)oxazole, triflate salt (16.5g, 0.04 mol) in dimethylformamide is treated at 10°C with 2-chloroacrylonitrile (5.25g, 0.06 mol) under a nitrogen atmosphere, warmed to room temperature, held at room temperature for 4 hours, and treated with a mixture of ethyl acetate and water. The phases are separated and the organic phase is washed with water and concentrated to give a wet solid residue. Flash column chromatography on silica gel, packed with 15% ethyl acetate in heptane and eluted with 20% ethyl acetate in heptane of the residue gives, on crystallization from ethyl acetate/heptane, the title product as a pale yellow solid, 7.lg (65% yield), mp 238.0°-241.0°C, identified by NMR analysis.

### Example 7

### Preparation of 5-Amino-4-(p-chlorophenyl)-3-methyl-2-(trifluoromethyl)-oxazolium fluoroborate

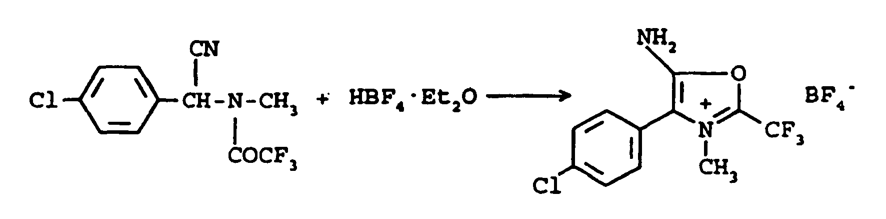

A solution of N-(*p*-chloro-α-cyanobenzyl-2,2,2-trifluoro-N-methylacetamide (13.8g, 0.05 mol) in toluene is treated with tetrafluoroboric acid, diethyl etherate (10.5g as is, 8.9g real, 0.055 mol) at room temperature. The title oxazolium salt precipitates out as a yellow solid, is filtered, and washed with dry ether and dried under a nitrogen atmosphere. The title product is identified by ¹⁹F NMR analysis (singlet at -60 ppm DMSO-D₆).

## Claims

1. A compound of formula I 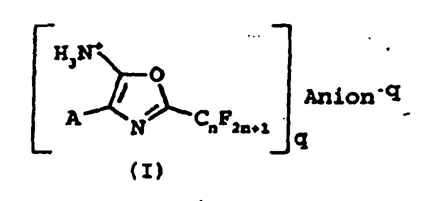 wherein n is an integer of 1, 2, 3, 4, 5, 6, 7 or 8;
q is an integer of 1, 2, or 3;
A is 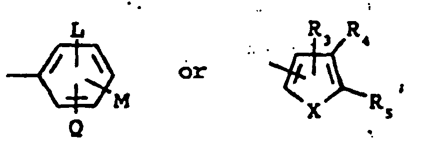 L is hydrogen or halogen;
M and Q are each independently halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl, M may also be hydrogen, or when M and Q are on adjacent positions they may be taken together with the carbon atoms to which they are attached to form a ring in which MQ represents the structure
-OCH₂O-, -OCF₂O- or -CH=CH-CH=CH-;
R₃, R₄, and R₅ are each independently hydrogen, halogen, NO₂, CHO or R₄ and R₅ may be taken together with the atoms to which they are attached to form a ring in which R₄R₅ is represented by the structure 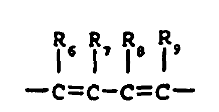 R₆, R₇, R₈ and R₉ are each independently hydrogen, halogen, CN or NO₂;
X is O or S; and
Anion^{-q} is a proton acceptor having a negative q charge; or
the tautomers thereof.

2. The compound according to claim 1 wherein q is 1; the anion^{-q} is selected from the group consisting of methanesulfonate, trifluoromethanesulfonate, benzenesulfonate, *p*-toluenesulfonate, naphthalenesulfonate, and tetrafluoroborate; n is 1 or 2; A is 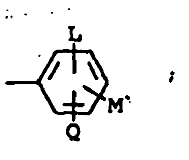 L is hydrogen or halogen and M and Q are each independently halogen or C₁-C₄haloalkyl and may also be hydrogen.

3. The compound according to claim 1:
5-amino-4-(*p*-chlorophenyl)-2-(trifluoromethyl)-oxazole, methanesulfonic acid salt;
5-amino-4-(*p*-chlorophenyl)-2-(trifluoromethyl) oxazole, *p*-toluenesulfonic acid salt;
5-amino-4-(3,5-dichlorophenyl)-2-(trifluoromethyl)-oxazole, trifluoromethanesulfonic acid salt;
5-amino-4-(3,5-dichlorophenyl)-2-(trifluoromethyl)-oxazole, methanesulfonic acid salt;
5-amino-4-[*p*-(α,α,α-trifluoro)tolyl]-2-(trifluoromethyl)oxazole, *p*-toluenesulfonic acid salt;
5-amino-4-(3,4,5-trichlorophenyl)-2-(trifluoromethyl)oxazole, *p* -toluenesulfonic acid salt;
5-amino-4-[*p*-(trifluoromethoxy)phenyl]-2-(trifluoromethyl)oxazole, trifluoromethanesulfonic acid salt; or
5-amino-4-[2-(3,4,5-trichloro)thienyl]-2-(trifluoromethyl)oxazole, methanesulfonic acid salt.

4. A compound of formula II 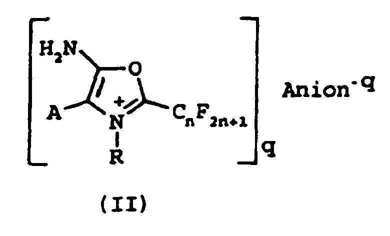 wherein n is an integer of 1, 2, 3, 4, 5, 6, 7 or 8;
q is an integer of 1, 2, or 3;
R is C₁-C₆alkyl optionally substituted with one C₁-C₄alkoxy or phenyl group;
A is 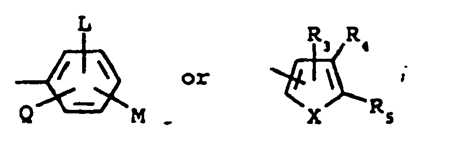 L is hydrogen or halogen;
M and Q are each independently halogen, CN, NO₂, C₁-C₄alkyl, C₁-C₄haloalkyl, C₁-C₄alkoxy, C₁-C₄haloalkoxy, C₂-C₄alkylthio, C₁-C₄alkylsulfinyl, M may also be hydrogen, or when M and Q are on adjacent positions they may be taken together with the carbon atoms to which they are attached to form a ring in which MQ represents the structure
-OCH₂O-, -OCF₂O- or -CH=CH-CH=CH-;
R₃, R₄, and R₅ are each independently hydrogen, halogen, NO₂, CHO or R₄ and R₅ may be taken together with the atoms to which they are attached to form a ring in which R₄R₅ is represented by the structure 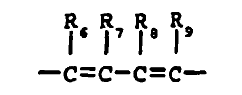 R₆, R₇, R₈ and R₉ are each independently hydrogen, halogen, CN or NO₂;
X is O or S; and
Anion^{-q} is a proton acceptor having a negative q charge; or
the tautomers thereof.

5. The compound according to claim 4 wherein q is 1; n is 1 or 2; R is methyl or benzyl; A is 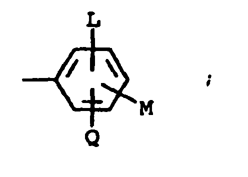 L is hydrogen or halogen and M and Q are each independently halogen or C₁-C₄haloalkyl, and M may also be hydrogen.

6. The compound according to claim 4
5-amino-4-(*p*-chlorophenyl)-3-methyl-2-(trifluoromethyl)oxazolium tetrafluoroborate;
5-amino-4-(*p*-bromophenyl)-3-methyl-2-(trifluoromethyl)oxazolium methanesulfonate;
5-amino-4-(3,5-dichlorophenyl)-3-benzyl-2-(trifluoromethyl)oxazolium *p*-chlorotoluenesulfonate;
5-amino-4-(3,4,5-trichlorophenyl)-3-isopropyl-2-(trifluoromethyl)oxazolium tetrafluoroborate;
5-amino-4-(*p*-chlorophenyl)-3-methyl-2-(trifluoromethyl)oxazolium bisulfate;
5-amino-4-[(α,α,α-trifluoro)tolyl]-3-benzyl-2-(trifluoromethyl)oxazolium *p*-chlorotoluenesulfonate;
5-amino-4-[*p*-(trifluoromethoxy)phenyl]-3-ethyl-2-(trifluoromethyl)oxazolium tetrafluoroborate; or
5-amino-4-[2-(3,4,5-trichloro)thienyl]-2-(trifluoromethyl)oxazolium methanesulfonate.

7. A method for the preparation of a compound of formula I or formula II 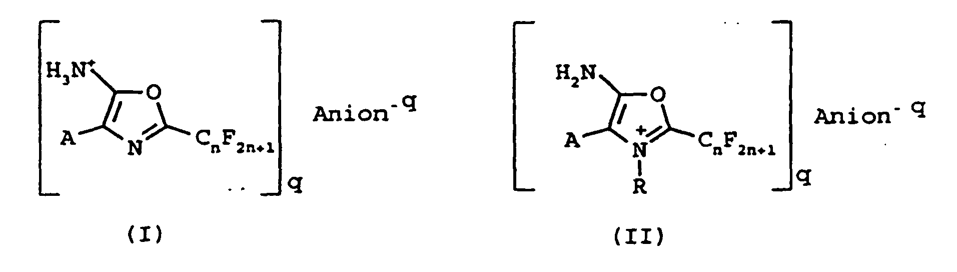 wherein n is an integer of 1, 2, 3, 4, 5, 6, 7 or 8;
q is an integer of 1, 2, or 3;
R is C₁-C₆alkyl optionally substituted with one C₁-C₄alkoxy or phenyl group;
A is 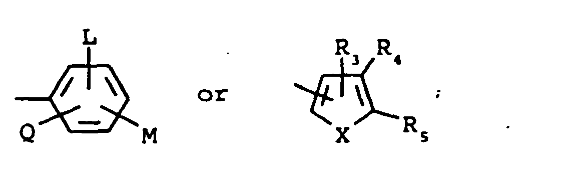 L is hydrogen or halogen;
M and Q are each independently halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl, M may also be hydrogen, or when M and Q are on adjacent positions they may be taken together with the carbon atoms to which they are attached to form a ring in which MQ represents the structure
-OCH₂O-, -OCF₂O- or -CH=CH-CH=CH-;
R₃, R₄, and R₅ are each independently hydrogen, halogen, NO₂, CHO or R₄ and R₅ may be taken together with the atoms to which they are attached to form a ring in which R₄R₅ is represented by the structure 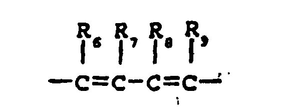 R₆, R₇, R₈ and R₉ are each independently hydrogen, halogen, CN or NO₂;
X is O or S; and
Anion^{-q} is a proton acceptor having a negative q charge; or
the tautomers thereof
which comprises reacting a compound of formula III 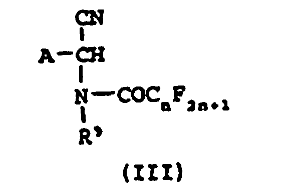 wherein n and A are described hereinabove and R' is H or R with at least one molar equivalent of an acid in the presence of a solvent and essentially in the absence of water.

8. The method according to claim 7 wherein the acid is selected from the group consisting of sulfuric acid, methanesulfonic acid, trifluoromethanesulfonic acid, benzenesulfonic acid, *p*-toluenesulfonic acid, naphthalenesulfonic acid, tetrafluoroboric acid, tetrafluoroboric acid etherate, and tetrafluoroboric acid alkanolate; and the solvent is selected from the group consisting of toluene, dimethylformamide, acetonitrile, propionitrile, *t*-butanol, and mixtures thereof.

9. A method for the preparation of a compound of formula IV 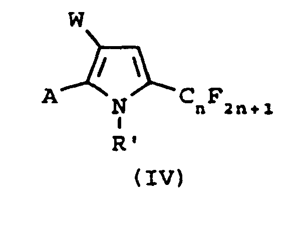 wherein W is CN, NO₂, COOR₁ or COR₂;
R₁ and R₂ are each independently C₁-C₄alkyl;
R' is hydrogen or R;
R is C₁-C₆alkyl optionally substituted with one C₁-C₄alkoxy or phenyl group;
n is an integer of 1, 2, 3, 4, 5, 6, 7 or 8;
A is 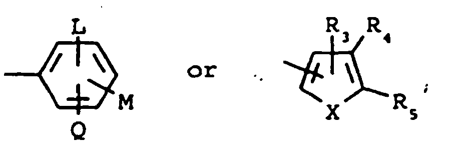 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure
-OCH₂O-, -OCF₂O- or -CH=CH-CH=CH-;
R₃, R₄, and R₅ are each independently hydrogen, halogen, NO₂, CHO or R₄ and R₅ may be taken together with the atoms to which they are attached to form a ring in which R₄R₅ is represented by the structure 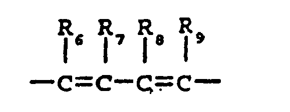 R₆, R₇, R₈ and R₉ are each independently hydrogen, halogen, CN or NO₂; and
X is O or S
which comprises reacting an intermediate compound of formula I or formula II 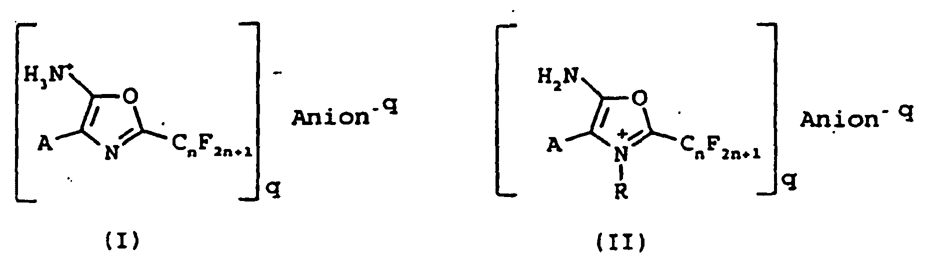 wherein n, A and R are as described hereinabove for formula IV and q is an integer of 1, 2, or 3 and Anion^{-q} is a proton acceptor having a negative q charge with at least q molar equivalents of a dienophile of formula V 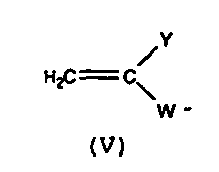 wherein W is as described hereinabove for formula IV and Y is hydrogen, Cl or Br with the proviso that when said intermediate compound is formula I then Y must be Cl or Br, in the presence of a solvent and essentially in the absence of water.

10. A method for the preparation of a compound of formula IVa 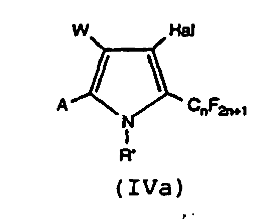 in which W, A, R' and n are as defined in claim 9 and Hal is a halogen atom, which comprises preparing the compound of formula IV as defined in claim 9 by the method defined in claim 9, and then halogenating the compound of formula IV to form the compound of formula IVa.

11. The method according to claim 9 or 10 wherein the solvent is an aromatic hydrocarbon, a halogenated aromatic hydrocarbon, an organic amide, a nitrile, an alkanol or mixtures thereof.

12. The method according to any of claims 9 to 11 in which W is CN, R' is methyl, n is 1 or 2, and A is phenyl optionally substituted with halogen, C₁-C₄haloalkyl, or C₁-C₄haloalkoxy.

13. The method according to any of claims 9 to 12 in which q is 1.

## Patentansprüche

1. Verbindung der Formel I 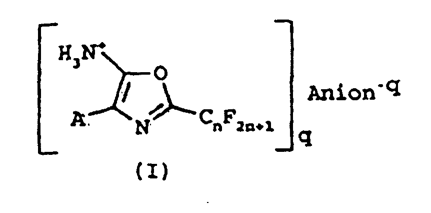 worin
n für 1, 2, 3, 4, 5, 6, 7 oder 8 steht;
q für 1, 2 oder 3 steht;
A für 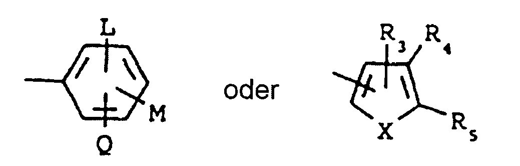
steht;
L Wasserstoff oder Halogen bedeutet;
M und Q unabhängig voneinander für Halogen, CN, NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl stehen, M auch für Wasserstoff stehen kann, oder für den Fall, dass M und Q benachbart sind, M und Q zusammen mit den Kohlenstoffatomen, an die sie gebunden sind, einen Ring bilden können, in dem MQ durch die Strukturformel
-OCH₂O- , -OCF₂O- oder -CH=CH-CH=CH-
dargestellt wird;
R₃, R₄ und R₅ unabhängig voneinander für Wasserstoff, Halogen, NO₂, CHO stehen, oder R₄ und R₅ zusammen mit den Atomen, an die sie gebunden sind, einen Ring bilden können, in dem R₄R₅ durch die Strukturformel 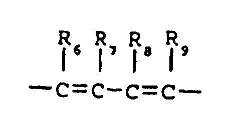
dargestellt wird;
R₆, R₇, R₈ und R₉ unabhängig voneinander für Wasserstoff, Halogen, CN oder NO₂ stehen;
X für O oder S steht; und
Anion^{-q} einen Protonenakzeptor mit einer negativen Ladung q bedeutet; und deren Tautomeren.

2. Verbindung gemäß Anspruch 1,
worin
q für 1 steht;
das Anion^{-q} unter Methansulfonat, Trifuormethansulfonat, Benzolsulfonat, p-Toluolsulfonat, Naphthalinsulfonat und Tetrafluorborat ausgewählt ist;
n für 1 oder 2 steht;
A für 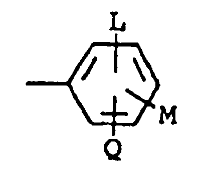
steht;
L Wasserstoff oder Halogen bedeutet; und
M und Q unabhängig voneinander für Halogen oder C₁-C₄-Halogenalkyl stehen, und M auch für Wasserstoff stehen kann.

3. Verbindung gemäß Anspruch 1:
5-Amino-4-(p-chlorphenyl)-2-(trifluormethyl)oxazol, Methansulfonsäuresalz;
5-Amino-4-(p-chlorphenyl)-2-(trifluormethyl)oxazol, p-Toluolsulfonsäuresalz;
5-Amino-4-(3,5-dichlorphenyl)-2-(trifluormethyl)oxazol, Trifluormethansulfonsäuresalz;
5-Amino-4-(3,5-dichlorphenyl)-2-(trifluormethyl)oxazol, Methansulfonsäuresalz;
5-Amino-4-[p-(α,α,α-trifluor)tolyl]-2-(trifluormethyl)oxazol, p-Toluolsulfonsäuresalz;
5-Amino-4-(3,4,5-trichlorphenyl)-2-(trifluormethyl)oxazol, p-Toluolsulfonsäuresalz;
5-Amino-4-[p-(trifluormethoxy)phenyl]-2-(trifluormethyl)oxazol, Trifluormethansulfonsäuresalz; oder
5-Amino-4-[2-(3,4,5-trichlor)thienyl]-2-(trifluormethyl)oxazol, Methansulfonsäuresalz.

4. Verbindung der Formel II 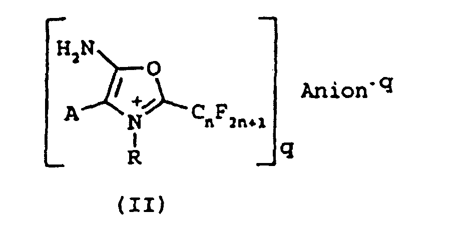 worin
n für 1, 2, 3, 4, 5, 6, 7 oder 8 steht;
q für 1, 2 oder 3 steht;
R für gegebenenfalls mit einem C₁-C₄-Alkoxy oder einer Phenylgruppe substituiertes C₁-C₆-Alkyl steht;
A für 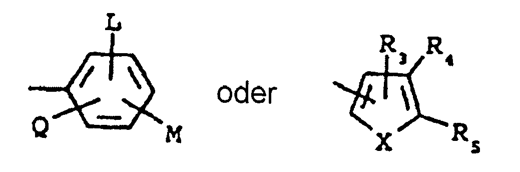
steht;
L Wasserstoff oder Halogen bedeutet;
M und Q unabhängig voneinander für Halogen, CN, NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl stehen, M auch für Wasserstoff stehen kann, oder für den Fall, dass M und Q benachbart sind, M und Q zusammen mit den Kohlenstoffatomen, an die sie gebunden sind, einen Ring bilden können, in dem MQ durch die Strukturformel
-OCH₂O- , -OCF₂O- oder -CH=CH-CH=CH-
dargestellt wird;
R₃, R₄ und R₅ unabhängig voneinander für Wasserstoff, Halogen, NO₂, CHO stehen, oder R₄ und R₅ zusammen mit den Atomen, an die sie gebunden sind, einen Ring bilden können, in dem R₄R₅ durch die Strukturformel 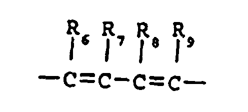
dargestellt wird;
R₆, R₇, R₈ und R₉ unabhängig voneinander für Wasserstoff, Halogen, CN oder NO₂ stehen;
X für O oder S steht; und
Anion^{-q} einen Protonenakzeptor mit einer negativen Ladung q bedeutet;
und deren Tautomeren.

5. Verbindung gemäß Anspruch 4,
worin
q für 1 steht;
n für 1 oder 2 steht;
R für Methyl oder Benzyl steht
A für 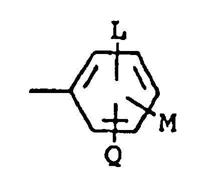
steht;
L Wasserstoff oder Halogen bedeutet; und
M und Q unabhängig voneinander für Halogen oder C₁-C₄-Halogenalkyl stehen, und M auch für Wasserstoff stehen kann.

6. Verbindung gemäß Anspruch 4:
5-Amino-4-(p-chlorphenyl)-3-methyl-2-(trifluormethyl)oxazolium-tetrafluorborat;
5-Amino-4-(p-bromphenyl)- 3-methyl-2-(trifluormethyl)oxazolium-methansulfonat;
5-Amino-4-(3,5-dichlorphenyl)-3-benzyl-2-(trifluormethyl)oxazolium-p-chlortoluolsulfonat;
5-Amino-4-(3,4,5-trichlorphenyl)-3-isopropyl-2-(trifluormethyl)oxazolium-tetrafluorborat;
5-Amino-4-(p-chlorphenyl)-3-methyl-2-(trifluormethyl)oxazolium-bisulfat;
5-Amino-4-[(α,α,α-trifluor)tolyl]-3-benzyl-2-(trifluormethyl)oxazolium-p-chlortoluolsulfonat;
5-Amino-4-[p-(trifluormethoxy)phenyl]-3-ethyl-2-(trifluormethyl)oxazolium-tetrafluorborat; oder
5-Amino-4-[2-(3,4,5-trichlor)thienyl]-2-(trifluormethyl)oxazolium-methansulfonat.

7. Verfahren zur Herstellung einer Verbindung der Formel I oder der Formel II 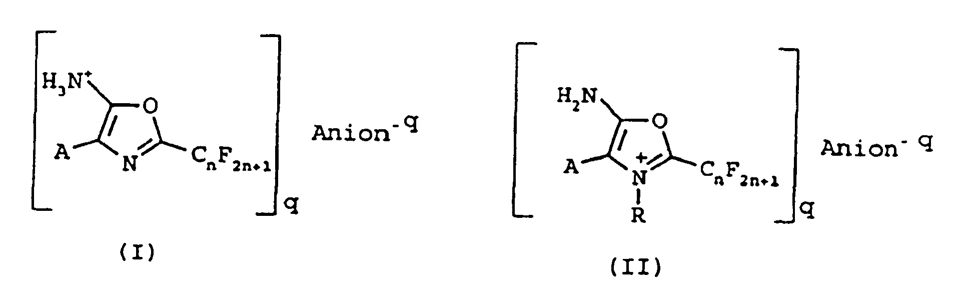 worin
n für 1, 2, 3, 4, 5, 6, 7 oder 8 steht;
q für 1, 2 oder 3 steht;
R für gegebenenfalls mit einem C₁-C₄-Alkoxy oder einer Phenylgruppe substituiertes C₁-C₆-Alkyl steht;
A für 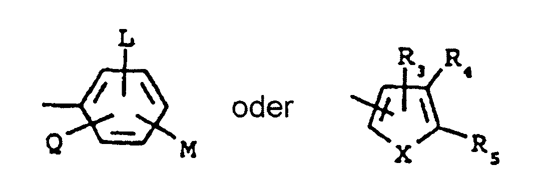
steht;
L Wasserstoff oder Halogen bedeutet;
M und Q unabhängig voneinander für Halogen, CN, NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl stehen, M auch für Wasserstoff stehen kann, oder für den Fall, dass M und Q benachbart sind, M und Q zusammen mit den Kohlenstoffatomen, an die sie gebunden sind, einen Ring bilden können, in dem MQ durch die Strukturformel
-OCH₂O- , -OCF₂O- oder -CH=CH-CH=CH-
dargestellt wird;
R₃, R₄ und R₅ unabhängig voneinander für Wasserstoff, Halogen, NO₂, CHO stehen, oder R₄ und R₅ zusammen mit den Atomen, an die sie gebunden sind, einen Ring bilden können, in dem R₄R₅ durch die Strukturformel 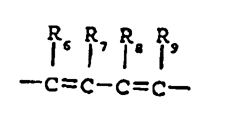
dargestellt wird;
R₆, R₇, R₈ und R₉ unabhängig voneinander für Wasserstoff, Halogen, CN oder NO₂ stehen;
X für O oder S steht; und
Anion^{-q} einen Protonenakzeptor mit einer negativen Ladung q bedeutet;
und deren Tautomeren,
umfassend die Umsetzung einer Verbindung der Formel III 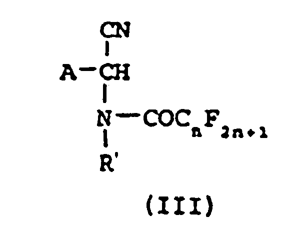 worin n und A wie oben bezeichnet sind, und R' für H oder R steht, mit mindestens einem Moläquivalent einer Säure in Gegenwart eines Lösungsmittels und im Wesentlichen in Abwesenheit von Wasser.

8. Verfahren gemäß Anspruch 7, wobei die Säure ausgewählt ist unter Schwefelsäure, Methansulfonsäure, Trifluormethansulfonsäure, Benzolsulfonsäure, p-Toluolsulfonsäure, Naphthalinsulfonsäure, Tetrafluorborsäure, Tetrafluorborsäure-Etherat, Tetrafluorborsäure-Alkanolat; und das Lösungsmittel ausgewählt ist unter Toluol, Dimethylformamid, Acetonitril, Propionitril, t-Butanol und deren Gemischen.

9. Verfahren zur Herstellung einer Verbindung der Formel IV 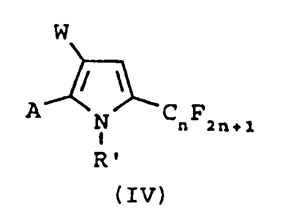 worin
W für CN, NO₂, COOR₁ oder COR₂ steht;
R₁ und R₂ unabhängig voneinander für C₁-C₄-Alkyl stehen;
R' für Wasserstoff oder R steht;
R für gegebenenfalls mit einem C₁-C₄-Alkoxy oder einer Phenylgruppe substituiertes C₁-C₆-Alkyl steht;
n für 1, 2, 3, 4, 5, 6, 7 oder 8 steht;
A für 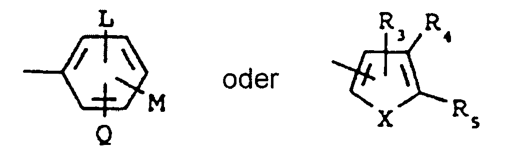
steht;
L Wasserstoff oder Halogen bedeutet;
M und Q unabhängig voneinander für Wasserstoff, Halogen, CN, NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl stehen, oder für den Fall, dass M und Q benachbart sind, M und Q zusammen mit den Kohlenstoffatomen, an die sie gebunden sind, einen Ring bilden können, in dem MQ durch die Strukturformel
-OCH₂O- , -OCF₂O- oder -CH=CH-CH=CH-
dargestellt wird;
R₃, R₄ und R₅ unabhängig voneinander für Wasserstoff, Halogen, NO₂, CHO stehen, oder R₄ und R₅ zusammen mit den Atomen, an die sie gebunden sind, einen Ring bilden können, in dem R₄R₅ durch die Strukturformel 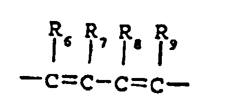
dargestellt wird;
R₆, R₇, R₈ und R₉ unabhängig voneinander für Wasserstoff, Halogen, CN oder NO₂ stehen;
X für O oder S steht;
umfassend die Umsetzung eines Zwischenproduktes der Formel I oder der Formel II 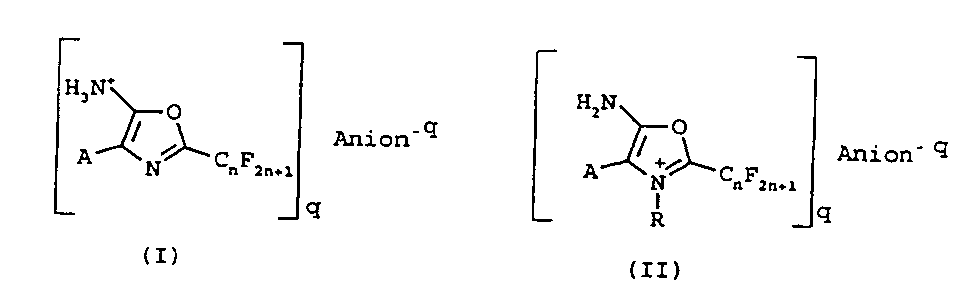 worin n, A und R wie oben für Formel IV bezeichnet sind, q für 1, 2 oder 3 steht und Anion^{-q} einen Protonenakzeptor mit einer negativen Ladung q bedeutet, mit mindestens q Moläquivalenten eines Dienophilens der Formel V 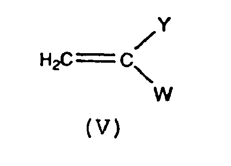 worin W wie oben für Formel IV bezeichnet ist und Y für Wasserstoff, Cl oder Br steht, mit der Maßgabe, dass, wenn dieses Zwischenprodukt Formel I entspricht, Y für Cl oder Br stehen muss,
in Gegenwart eines Lösungsmittels und im Wesentlichen in Abwesenheit von Wasser.

10. Verfahren zur Herstellung einer Verbindung der Formel IVa 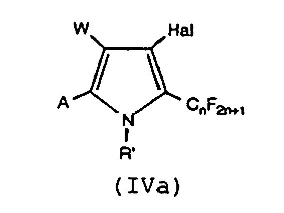 worin W, A, R' und n wie in Anspruch 9 definiert sind und Hal für ein Halogenatom steht, umfassend die Herstellung der in Anspruch 9 definierten Verbindung der Formel IV nach dem in Anspruch 9 definierten Verfahren und die anschließende Halogenierung der Verbindung der Formel IV, wobei man eine Verbindung der Formel IVa erhält.

11. Verfahren nach Anspruch 9 oder 10, wobei das Lösungsmittel ein aromatischer Kohlenwasserstoff, ein halogenierter aromatischer Kohlenwasserstoff, ein organisches Amid, ein Nitril, ein Alkanol oder ein Gemisch davon ist.

12. Verfahren nach einem der Ansprüche 9 bis 11, wobei W für CN, R' für Methyl, n für 1 oder 2 und A für gegebenenfalls mit Halogen, C₁-C₄-Halogenalkyl oder C₁-C₄-Halogenalkoxy substituiertes Phenyl steht.

13. Verfahren nach einem der Ansprüche 9 bis 12, wobei q für 1 steht.

## Revendications

1. Composé de formule 1 : 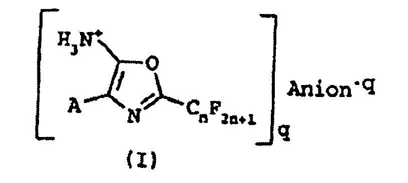 dans laquelle n est un entier valant 1, 2, 3, 4, 5, 6, 7 ou 8;
q est un entier valant 1, 2, ou 3;
A est 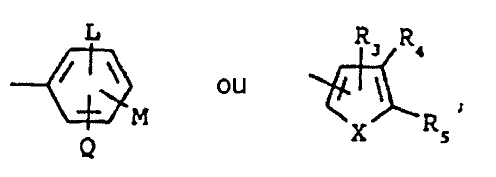 L représente l'hydrogène ou un halogène;
M et Q représentent chacun, indépendamment, un halogène, un groupement CN, NO₂, alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄, M peut également représenter l'hydrogène ou, lorsque M et Q se trouvent en positions adjacentes, ils peuvent être pris conjointement avec les atomes de carbone auxquels ils sont liés pour former un cycle dans lequel MQ représente la structure :
-OCH₂O-, -OCF₂O- ou -CH=CH-CH=CH-;
R₃, R₄ et R₅ représentent chacun, indépendamment, de l'hydrogène, un halogène, NO₂, CHO ou R₄ et R₅ peuvent être pris conjointement avec les atomes auxquels ils sont liés pour former un cycle dans lequel R₄R₅ est représenté par la structure : 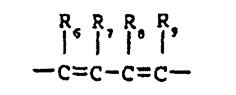 où R₆, R₇, R₈ et R₉ représentent chacun, indépendamment, l'hydrogène, un halogène, CN ou NO₂;
X est O ou S; et
anion^{-q} est un accepteur de protons ayant une charge q négative; ou ses tautomères.

2. Composé selon la revendication 1, dans lequel q vaut 1; l'anion^{-q} est choisi dans le groupe constitué du méthanesulfonate, du trifluorométhanesulfonate, du benzènesulfonate, du p-toluènesulfonate, du naphtalènesulfonate et du tétrafluoroborate; n vaut 1 ou 2; A est 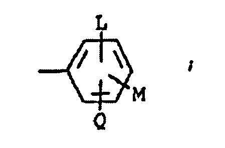 L est de l'hydrogène ou un halogène et M et Q représentent chacun, indépendamment, un halogène ou un halogénoalkyle en C₁-C₄ et M peut également être de l'hydrogène.

3. Composé selon la revendication 1 :
sel d'acide méthanesulfonique du 5-amino-4-(p-chlorophényl)-2-(trifluorométhyl)oxazole;
sel d'acide p-toluènesulfonique du 5-amino-4-(p-chlorophényl)-2-(trifluorométhyl)oxazole;
sel d'acide trifluorométhanesulfonique du 5-amino-4-(3,5-dichlorophényl)-2-(trifluorométhyl)-oxazole;
sel d'acide méthanesulfonique du 5-amino-4-(3,5-dichlorophényl)-2-(trifluorométhyl)oxazole;
sel d'acide p-toluènesulfonique du 5-amino-4-[p-(α,α,α-trifluoro)tolyl]-2(trifluorométhyl)oxazole;
sel d'acide p-toluènesulfonique du 5-amino-4-(3,4,5-trichlorophényl)-2-(trifluorométhyl)oxazole;
sel d'acide trifluorométhanesulfonique du 5-amino-4-[p-(trifluorométhoxy)phényl)-2-(trifluorométhyl)oxazole; ou
sel d'acide méthanesulfonique du 5-amino-4-[2-(3,4,5-trichloro)thiényl]-2-(trifluorométhyl)oxazole.

4. Composé de formule II : 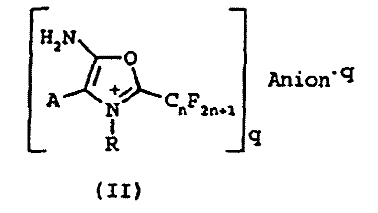 dans laquelle n est un entier valant 1, 2, 3, 4, 5, 6, 7 ou 8;
q est un entier valant 1, 2, ou 3;
R est un alkyle en C₁-C₆ le cas échéant substitué par un groupement alcoxy en C₁-C₄ ou phényle;
A est 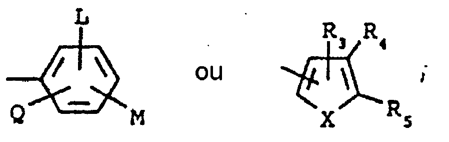 L représente l'hydrogène ou un halogène;
M et Q représentent chacun, indépendamment, un halogène, un groupement CN, NO₂, alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄, M peut également représenter l'hydrogène ou, lorsque M et Q se trouvent en positions adjacentes, ils peuvent être pris conjointement avec les atomes de carbone auxquels ils sont liés pour former un cycle dans lequel MQ représente la structure :
-OCH₂O-, -OCF₂O- ou -CH=CH-CH=CH-;
R₃, R₄ et R₅ représentent chacun, indépendamment, l'hydrogène, un halogène, NO₂, CHO ou R₄ et R₅ peuvent être pris conjointement avec les atomes auxquels ils sont liés pour former un cycle dans lequel R₄R₅ est représenté par la structure : 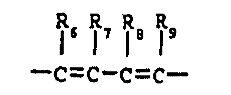 où R₆, R₇, R₈ et R₉ représentent chacun, indépendamment, l'hydrogène, un halogène, CN ou NO₂;
X est O ou S; et
anion^{-q} est un accepteur de protons ayant une charge q négative;
ou ses tautomères.

5. Composé selon la revendication 4, dans lequel q vaut 1; n vaut 1 ou 2; R est un méthyle ou un benzyle; A est 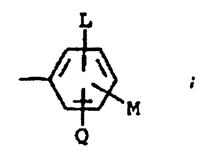 L est l'hydrogène ou un halogène et M et Q représentent chacun, indépendamment, un halogène ou un halogénoalkyle en C₁-C₄ et M peut également représenter l'hydrogène.

6. Composé selon la revendication 4 :
tétrafluoroborate du 5-amino-4-(p-chlorophényl)-3-méthyl-2-(trifluorométhyl)oxazolium;
méthanesulfonate du 5-amino-4-(p-bromophényl)-3-méthyl-2-(trifluorométhyl)oxazolium;
p-chlorotoluènesulfonate du 5-amino-4-(3,5-dichlorophényl)-3-benzyl-2-(trifluorométhyl)oxazolium;
tétrafluoroborate du 5-amino-4-(3,4,5-trichlorophényl)-3-isopropyl-2-(trifluorométhyl)-oxazolium;
bisulfate du 5-amino-4-(p-chlorophényl)-3-méthyl-2-(trifluorométhyl)oxazolium;
p-chlorotoluènesulfonate du 5-amino-4-[(α,α,α-trifluoro)tolyl]-3-benzyl-2-(trifluorométhyl)oxazolium;
tétrafluoroborate du 5-amino-4-[p-(trifluorométhoxy)phényl]-3-éthyl-2-(trifluorométhyl)oxazolium; ou
méthanesulfonate du 5-amino-4- [2- (3,4,5-trichloro)thiényl]-2-(trifluorométhyl)oxazolium.

7. Procédé pour la préparation d'un composé de formule I ou de formule II : 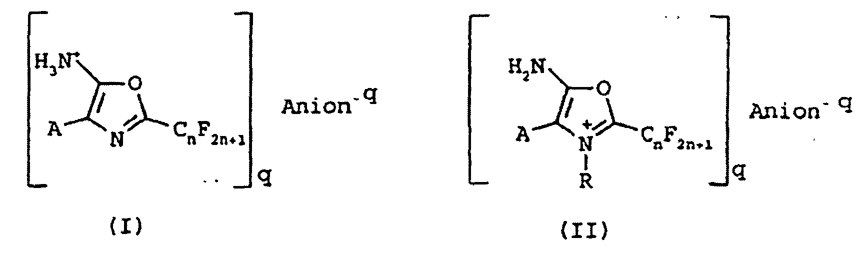 dans lesquelles n est un entier valant 1, 2, 3, 4, 5, 6, 7 ou 8;
q est un entier valant 1, 2, ou 3;
R est un alkyle en C₁-C₆ le cas échéant substitué par un groupement alcoxy en C₁-C₄ ou phényle;
A est 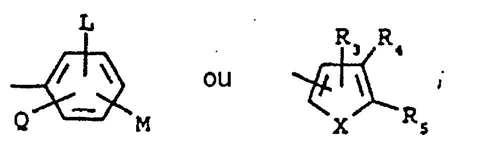 L représente l'hydrogène ou un halogène;
M et Q représentent chacun, indépendamment, un halogène, un groupement CN, NO₂, alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄, M peut également représenter l'hydrogène ou, lorsque M et Q se trouvent en positions adjacentes, ils peuvent être pris conjointement avec les atomes de carbone auxquels ils sont liés pour former un cycle dans lequel MQ représente la structure :
-OCH₂O-, -OCF₂O- ou -CH=CH-CH=CH-;
R₃, R₄ et R₅ représentent chacun, indépendamment, l'hydrogène, un halogène, NO₂, CHO ou R₄ et R₅ peuvent être pris conjointement avec les atomes auxquels ils sont liés pour former un cycle dans lequel R₄R₅ est représenté par la structure : 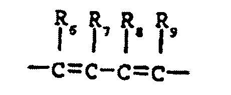 où R₆, R₇, R₈ et R₉ représentent chacun, indépendamment, l'hydrogène, un halogène, CN ou NO₂;
X est O ou S; et
anion^{-q} est un accepteur de protons ayant une charge q négative;
ou ses tautomères,
qui consiste à faire réagir un composé de formule III : 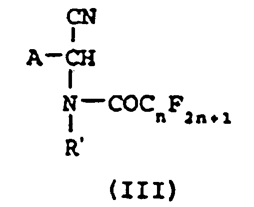 dans laquelle n et A sont décrits ci-dessus et R' représente H ou R avec au moins un équivalent molaire d'un acide en présence d'un solvant et essentiellement en l'absence d'eau.

8. Procédé selon la revendication 7, dans lequel l'acide est choisi dans le groupe constitué de l'acide sulfurique, de l'acide méthanesulfonique, de l'acide trifluorométhanesulfonique, de l'acide benzène-sulfonique, de l'acide p-toluènesulfonique, de l'acide naphtalènesulfonique, de l'acide tétrafluoroborique, de l'éthérate d'acide tétrafluoroborique et de l'alcanolate d'acide tétrafluoroborique; et le solvant est choisi dans le groupe constitué du toluène, du diméthylformamide, de l'acétonitrile, du propionitrile, du t-butanol et de leurs mélanges.

9. Procédé pour la préparation d'un composé de formule IV : 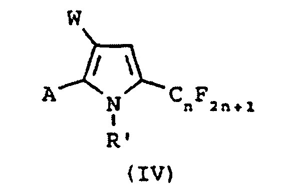 dans laquelle W est CN, NO₂, COOR₁ ou COR₂;
R₁ et R₂ représentent chacun, indépendamment, un alkyle en C₁-C₄;
R' est l'hydrogène ou R;
R est un alkyle en C₁-C₆ le cas échéant substitué par un groupement alcoxy en C₁-C₄ ou phényle;
n est un entier valant 1, 2, 3, 4, 5, 6, 7 ou 8;
A est 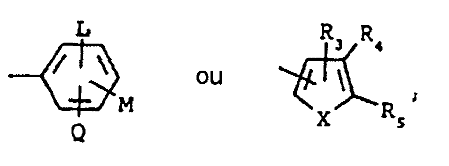 L est l'hydrogène ou un halogène;
M et Q représentent chacun, indépendamment, l'hydrogène, un halogène, un groupement CN, NO₂, alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄ ou, si M et Q se trouvent en positions adjacentes, ils peuvent être pris conjointement avec les atomes de carbone auxquels ils sont liés pour former un cycle dans lequel MQ représente la structure :
-OCH₂O-, -OCF₂O- ou -CH=CH-CH=CH-;
R₃, R₄ et R₅ représentent chacun, indépendamment, l'hydrogène, un halogène, NO₂, CHO ou R₄ et R₅ peuvent être pris conjointement avec les atomes auxquels ils sont liés pour former un cycle dans lequel R₄R₅ est représenté par la structure : 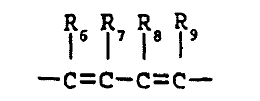 où R₆, R₇, R₈ et R₉ représentent chacun, indépendamment, l'hydrogène, un halogène, CN ou NO₂; et
X est O ou S;
qui consiste à faire réagir un composé intermédiaire de formule I ou de formule II : 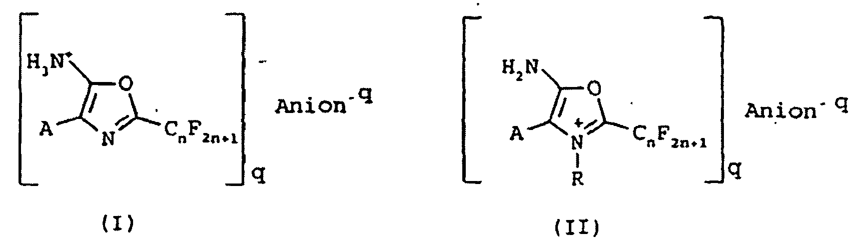 dans lesquelles n, A et R sont tels que décrits ci-dessus pour la formule IV et q est un entier valant 1, 2 ou 3 et anion^{-q} est un accepteur de protons ayant une charge q négative avec au moins q équivalents molaires d'un diénophile de formule V : 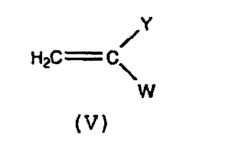 dans laquelle W est tel que décrit ci-dessus pour la formule IV et Y représente l'hydrogène, Cl ou Br à condition que, lorsque ledit composé intermédiaire répond à la formule I, Y soit Cl ou Br, en présence d'un solvant et essentiellement en l'absence d'eau.

10. Procédé pour la préparation d'un composé de formule IVa 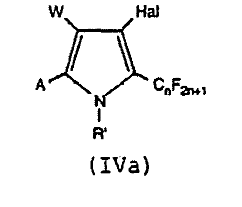 dans laquelle W, A, R' et n sont tels que définis dans la revendication 9 et Hal est un halogène, qui consiste à préparer le composé de formule IV de la manière définie dans la revendication 9 selon le procédé défini dans la revendication 9, puis à effectuer l'halogénation du composé de formule IV pour former le composé de formule IVa.

11. Procédé selon la revendication 9 ou 10, dans lequel le solvant est un hydrocarbure aromatique, un hydrocarbure aromatique halogéné, un amide organique, un nitrile, un alcanol ou leurs mélanges.

12. Procédé selon l'une quelconque des revendications 9 à 11, dans lequel W est CN, R' est un méthyle, n vaut 1 ou 2 et A est un phényle le cas échéant substitué par un halogène, un halogénoalkyle en C₁-C₄ ou un halogénoalcoxy en C₁-C₄.

13. Procédé selon l'une quelconque des revendications 9 à 12 dans lequel q vaut 1.
